# EUROPEAN PATENT APPLICATION

(11) **EP 0 705 607 A2**
(43) Date of publication of application: **10.04.1996**
(21) Application number: 95115109.1
(22) Date of filing: 26.09.1995
(51) Int. Cl.: A61K 31/55, A61K 31/395, A61K 31/553, A61K 31/554, A61P 3/06

(54) **Benzoxazepine and benzothiazepine derivatives useful as antihypertriglyceridemic agents**

(30) Priority: 07.10.1994 JP 19940244136
(71) Applicant: TAKEDA CHEMICAL INDUSTRIES, LTD., Chuo-ku, Osaka 541 (JP)
(72) Inventor: Sugiyama, Yasuo, Kawanishi, Hyogo 666-01 (JP); Yukimasa, Hidefumi, Nara 630 (JP)
(74) Representative: von Kreisler, Alek, Dipl.-Chem., et al

(57) **Abstract**

An antihypertriglyceridemic composition comprising a compound of formula (I) 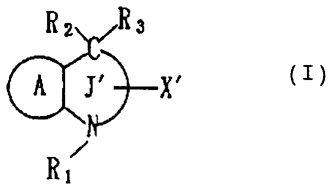 wherein R₁ represents a hydrogen atom or a hydrocarbon group that may be substituted; R₂ and R₃ may be the same or different and each represents a hydrogen atom, a hydrocarbon group that may be substituted, or a heterocyclic group that may be substituted; X' represents a substituent comprising a carboxyl group that may be esterified, a carbamoyl group that may be substituted, a hydroxyl group that may be substituted, an amino group that may be substituted, or an optionally substituted heterocyclic group having a hydrogen atom that may be deprotonated; ring A represents a benzene ring that may be substituted or a heterocyclic ring that may be substituted; ring J' represents a 7- or 8-membered heterocyclic ring having at most 3 hetero-atoms as ring constituent members; and ring J' may have a further substituent in addition to R₁, R₂, R₃ and X', or a pharmacologically acceptable salt thereof.

The composition has a plasma triglyceride concentration-lowering activity, and therefore is useful for the prophylaxis or treatment of hyperlipemia, such as hypertriglyceridemia.

## Description

### Field of the Invention

The present invention relates to an antihypertriglyceridemic (plasma triglyceride concentration-lowering) composition useful for the prophylaxis or treatment of hyperlipemia.

### Background of the Invention

An abnormal elevation of the serum (plasma) lipid level is known as hyperlipidemia or hyperlipemia. While the serum (plasma) lipid includes cholesterol (cholesterol esters, free cholesterol), phospholipids (lecithin, sphyngomyelin, etc.), triglycerides (neutral fat), free fatty acids, and other sterols, it is an elevation of cholesterol and triglyceride concentrations, in particular, that is of clinical importance [Common Disease Series No. 19, Hyperlipemia, ed. Haruo Nakamura, October 10, 1991, Nankodo].

Therefore, adequate control of blood lipid concentration is of vital importance for the prophylaxis and therapy of atherosclerotic diseases represented by ischemic heart disease, cerebral infarction and so forth. Moreover, hypertriglyceridemia is suspected to be complicated by a disorder of the pancreas.

As blood cholesterol-lowering agents, drugs adapted to bind bile acid and inhibit its absorption, such as cholestyramine, colestipol, etc. (e.g. US Patent 4027009), compounds adapted to inhibit acylcoenzyme A cholesterol acyltransferase (ACAT) and control intestinal absorption of cholesterol, such as melinamide (French Patent 1476569), and drugs which inhibit biosynthesis of cholesterol are attracting attention. As such cholesterol biosynthesis inhibitors, lovastatin (US Patent 4231938), simvastatin (US Patent 4444784) and pravastatin (US Patent 4346227), which inhibit 3-hydroxy3-methylglutaryl-coenzyme A (HMG-CoA) reductase, in particular, have been put to clinical use. However, inhibition of HMG-CoA reductase leads not only to inhibition of cholesterol biosynthesis but also to inhibition of the biosynthesis of other vital factors such as ubiquinones, dolichols and heme A, there are concerns about the risk of associated side effects.

As drugs for lowering blood triglyceride concentrations, fibric acid-related compounds such as clofibrate (U. K. Patent 860303) and phenofibrate (German Patent 2250327) are available for medicinal use but administration of these compounds in combination with statin compounds is contraindicated because of hepatotoxicity.

Hyperlipemia is also known as hyperlipoproteinemia and, by the kind of lipoprotein, has been classified into the following six types (WHO Classification).
Type I: Hyperchylomicronemia characterized by elevation of the concentration of chylomicrons
IIa: Hyper-LDLdiseases (hypercholesterolemia) characterized by elevation of low-density lipoprotein (LDL)
IIb: Compound hyperlipemia characterized by elevation of LDL and very-low-density lipoprotein (VLDL)
III: Abnormal β-lipoproteinemia characterized by the presence of f3-very-low-density lipoprotein (βVLDL)
IV: Endogenous triglyceridemia characterized by elevation of VLDL
V: Mixed hyperlipemia characterized by elevation of the concentration of VLDL and chylomicrons

### Summary of the Invention

The present invention provides an antihypertriglyceridemic medicine of value for the prophylaxis or treatment of hyperlipemia, especially an antihypertriglyceridemic medicine exhibiting its activity through new action.

### Detailed Description of the Invention

The inventors of the present invention who explored the above state of the art and did much research, discovered that a class of fused-cyclic compounds has an excellent plasma triglyceride-lowering activity and have arrived at the present invention.

The present invention, therefore, relates to:
(1) an antihypertriglyceridemic composition comprising a compound of the formula (I) 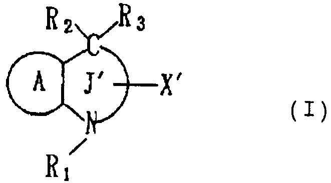 wherein R₁ represents a hydrogen atom or a hydrocarbon group that may be substituted; R₂ and R₃ may be the same or different and each represents a hydrogen atom, a hydrocarbon group that may be substituted, or a heterocyclic group that may be substituted; X' represents a substituent comprising a carboxyl group that may be esterified, a carbamoyl group that may be substituted, a hydroxyl group that may be substituted, an amino group that may be substituted, or an optionally substituted heterocyclic group having a hydrogen atom that may be deprotonated; ring A represents a benzene ring that may be substituted or a heterocyclic ring that may be substituted; ring J' represents a 7- or 8-membered heterocyclic ring having at most 3 hetero-atoms as ring constituent members; and ring J' may have a further substituent in addition to R₁, R₂, R₃ and X', or a pharmacologically acceptable salt thereof; among others,
(2) an antihypertriglyceridemic composition of (1) wherein R₂ and R₃ are independently a hydrogen atom, an alkyl group that may be substituted, a phenyl group that may be substituted, or an aromatic heterocyclic group that may be substituted.
(3) an antihypertriglyceridemic composition comprising a compound of the formula (I') 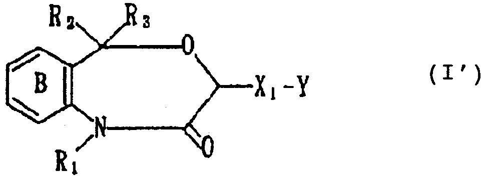 wherein R₁ represents a hydrogen atom or a hydrocarbon group that may be substituted; R₂ and R₃ may be the same or different and each represents a hydrogen atom, a hydrocarbon group that may be substituted, or a heterocyclic group that may be substituted; X₁ represents a bond or a divalent atomic chain; Y represents a carboxyl group that may be esterified, a carbamoyl group that may be substituted, a hydroxyl group that may be substituted, an amino group that may be substituted, or an optionally substituted heterocyclic group having a hydrogen atom that may be deprotonated and ring B represents a benzene ring that may be substituted, or a pharmacologically acceptable salt thereof, and
(4) the antihypertriglyceridemic composition (1), (2) or (3) for the prophylaxis or treatment of hyperlipemia particularly hypertriglyceridemia.

Referring to formulas (I) and (I'), the hydrocarbon group for said "hydrocarbon group that may be substituted" as designated by R₁ includes aliphatic acyclic hydrocarbon groups, alicyclic hydrocarbon groups, and aryl groups and is preferably an aliphatic acyclic hydrocarbon group.

The aliphatic acyclic hydrocarbon group for said hydrocarbon group includes straight-chain or branched aliphatic hydrocarbon groups such as alkyl, alkenyl and alkinyl groups and is preferably a branched alkyl group. The alkyl group mentioned just above includes C₁₋₇ alkyl groups such as methyl, ethyl, n-propyl, isopropyl, n-butyl, isobutyl, sec-butyl, tert-butyl, n-pentyl, isopentyl, neopentyl, 1-methylpropyl, n-hexyl, isohexyl, 1,1-dimethylbutyl, 2,2-dimethylbutyl, 3,3-dimethylbutyl, 3,3-dimethylpropyl, 2-ethylbutyl, n-heptyl, etc. and is preferably a C₃₋₅ alkyl group such as n-propyl, isopropyl, isobutyl, neopentyl, etc. Particularly preferred are isobutyl and neopentyl. The alkenyl group includes C₂₋₆ alkenyl groups such as vinyl, allyl, isopropenyl, 2-methylallyl, 1-propenyl, 2-methyl-1-propenyl, 2-methyl-2-propenyl, 1-butenyl, 2-butenyl, 3-butenyl, 2-ethyl-1-butenyl, 2-methyl-2-butenyl, 3-methyl-2-butenyl, 1-pentenyl, 2-pentenyl, 3-pentenyl, 4-pentenyl, 4-methyl-3-pentenyl, 1-hexenyl, 2-hexenyl, 3-hexenyl, 4-hexenyl, 5-hexenyl and is preferably vinyl, allyl, isopropenyl, 2-methylallyl, 2-methyl-1-propenyl, 2-methyl-2-propenyl, and 3-methyl-2-butenyl, among others. The alkinyl group includes C₂₋₆ alkinyl groups such as ethinyl, 1-propinyl, 2-propinyl, 1-butinyl, 2-butinyl, 3-butinyl, 1-pentinyl, 2-pentinyl, 3-pentinyl, 4-pentinyl, 1-hexinyl, 2-hexinyl, 3-hexinyl, 4-hexinyl, 5-hexinyl, etc. and is preferably ethinyl, 1-propinyl or 2-propinyl.

The alicyclic hydrocarbon group for said hydrocarbon group includes saturated or unsaturated alicyclic hydrocarbon groups such as cycloalkyl, cycloalkenyl, cycloalkadienyl, etc. The cycloalkyl mentioned just above includes C₃₋₉ cycloalkyl groups such as cyclopropyl, cyclobutyl, cyclopentyl, cyclohexyl, cycloheptyl, cyclooctyl, cyclononyl, etc. and is preferably a C₃₋₆ cycloalkyl group such as cyclopropyl, cyclobutyl, cyclopentyl or cyclohexyl. The cycloalkenyl includes C₃₋₆ cycloalkenyl groups such as 2-cyclopenten-1-yl, 3-cyclopenten-1-yl, 2-cyclohexen-1-yl, 3-cyclohexen-1-yl, 1-cyclobuten-1-yl, and 1-cyclopenten-1-yl, among others. The cycloalkadienyl includes C₃₋₆ cycloalkadienyl such as 2,4-cyclopentadien-1-yl, 2,4-cyclohexadien-1-yl, and 2,5-cyclohexadien-1-yl, among others.

The aryl group for said hydrocarbon group includes C₆₋₁₆ monocyclic and fused polycyclic aromatic hydrocarbon groups such as phenyl, naphthyl, anthryl, phenanthryl, acenaphthylenyl, etc. and is preferably a C₆₋₁₀ aryl group exemplified by phenyl, 1-naphthyl or 2-naphtyl.

The substitutent for said hydrocarbon group that may be substituted as designated by R₁ includes aryl that may be substituted, cycloalkyl or cycloalkenyl that may be substituted, heterocyclic groups that may be substituted, amino that may be substituted, hydroxyl that may be substituted, thiol that may be substituted, halogen (e.g. fluorine, chlorine, bromine, iodine), oxo, etc. and this hydrocarbon group may be substituted by 1 to 5, preferably 1 to 3 of such substituents in substitutable positions. The aryl group of said aryl that may be substituted includes C₆₋₁₆ aryl groups such as phenyl, naphthyl, anthryl, phenanthryl, acenaphthylenyl, etc. and is preferably a C₆₋₁₀ aryl group such as phenyl, 1-naphtyl or 2-naphthyl. The substituent for said aryl that may be substituted includes C₁₋₃ alkoxy (e.g. methoxy, ethoxy, propoxy, etc.), halogen (e.g. fluorine, chlorine, bromine, iodine) and C₁₋₃ alkyl (e.g. methyl, ethyl, propyl, etc.), and said aryl group may be substituted by 1 to 2 of such substituents. The cycloalkyl group of said cycloalkyl that may be substituted includes C₃₋₇ cycloalkyl groups such as cyclopropyl cyclobutyl, cyclopentyl, cyclohexyl and cycloheptyl. The substituent for this cycloalkyl that may be substituted may be similar, in kind and number, to the substituent mentioned for said aryl that may be substituted. The cycloalkenyl group of said cycloalkenyl that may be substituted includes C₃₋₆ cycloalkenyl groups such as cyclopropenyl, cyclobutenyl, cyclopentenyl, cyclohexenyl, etc. The substituent for this cycloalkenyl that may be substituted may be similar, in kind and number, to the substituent mentioned for said aryl that may be substituted. The heterocyclic group of said heterocyclic group that may be substituted includes aromatic heterocyclic groups and saturated or unsaturated nonaromatic heterocyclic (heteroaliphatic) groups containing at least one hetero-atom, preferably 1 to 4 hetero-atom(s) selected from among oxygen, sulfur and nitrogen as the ring constituent member (ring atom) and is preferably a aromatic heterocyclic group. The aromatic heterocyclic group mentioned above includes 5- to 8-membered aromatic monocyclic heterocyclic groups (e.g. furyl, thienyl, pyrrolyl, oxazolyl, isooxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, triazinyl, etc.) and fused aromatic heterocyclic groups composed of 2 to 3 5- to 8- membered rings (e.g. benzofuranyl, isobenzofuranyl, benzo[b]thienyl, indolyl, isoindolyl, 1H-indazolyl, benzimidazolyl, benzoxazolyl, 1,2-benzisooxazolyl, benzothiazolyl, 1,2-benzisothiazolyl, 1H-benzotriazolyl, quinolyl, isoquinolyl, cinnolinyl, quinazolinyl, quinoxalinyl, phthalazinyl, naphthyridinyl, purinyl, pteridinyl, carbazolyl, α-carbolinyl, β-carbolinyl, γ-carbolinyl, acridinyl, phenoxazinyl, phenothiazinyl, phenazinyl, phenoxathiinyl, thianthrenyl, phenanthridinyl, phenanthrolinyl, indolizinyl, pyrrolo[1,2-b]pyridazinyl, pyrazolo[1,5-a]pyridyl, imidazo[1,2-b]pyridyl, imidazo[1,5-a]pyridyl, imidazo[1,2-b]pyridazinyl, imidazo[1,2-a]pyrimidinyl, 1,2,4-triazolo[4,3-a]pyridyl, 1,2,4-triazolo[4,3-b]pyridazinyl, etc.). Particularly preferred are 5- or 6-membered aromatic monocyclic heterocyclic groups such as furyl, thienyl, indolyl, isoindolyl, pyrazinyl, pyridyl, and pyrimidinyl. The nonaromatic heterocyclic group (heteroaliphatic group) mentioned above includes 4- to 8-membered nonaromatic hetero cyclic groups such as oxiranyl, azetidinyl, oxetanyl, thietanyl, pyrrolidinyl, tetrahydrofuryl, thiolanyl, piperidyl, tetrahydropyranyl, morpholinyl, thiomorpholinyl and piperazinyl, among others. Said heterocyclic group that may be substituted may have 1 to 4 substituent(s) preferably 1 to 2 substituent(s), which are exemplified by C₁₋₃ alkyl groups (e.g. methyl, ethyl, propyl, etc.), among others. The substituent for said amino that may be substituted (mono- or di- substituted amino being included), for said hydroxyl that may be substituted, or for said thiol that may be substituted includes but is not limited to lower(C₁₋₃)alkyl groups (e,g. methyl, ethyl, propyl, etc.). Where the hydrocarbon moiety of said hydrocarbon group that may be substituted as designated by R₁ is an alicyclic hydrocarbon group or an aryl group, the substituent further includes (C₁₋₃ alkyl groups (e.g. methyl, ethyl, propyl, etc.).

As mentioned above, examples of the substituent for R₁ include oxo group and accordingly R₁ includes acyl groups derived from carboxylic acids as such hydrocarbon groups substituted by oxo group. Such examples of R₁ are C₁₋₆ acyl groups that may be substituted (e.g. formyl, acetyl, propionyl, butyryl, isobutyryl, valeryl, isovaleryl, pivaloyl, hexanoyl, dimethylacetyl, trimethylacetyl, etc.). Such acyl groups may have 1 to 5 substituents in any of substitutable positions, halogen (e.g. fluorine, chlorine, bromine) being typical of such substituents.

Referring to formulas (I) and (I'), as the "hydrocarbon groups that may be substituted" for R₂ and R₃, mention is made of the hydrocarbon groups as mentioned for the "hydrocarbon groups that may be substituted" represented by R₁, provided that the alkyl groups, aryl groups and their substitutents are preferably as mentioned below. That is, the alkyl group of said alkyl that may be substituted for R₂ and R₃ includes lower(C₁₋₆)alkyl groups (e.g. methyl, ethyl, n-propyl, isopropyl, butyl, isobutyl, sec-butyl, tert-butyl, pentyl, isopentyl, neopentyl, hexyl, isohexyl, etc.) and is preferably a C₁₋₄ alkyl group such as methyl, ethyl, propyl, isopropyl, butyl, t-butyl, etc., and the said alkyl that may be substituted may have 1 to 4 substituents, which are exemplified by halogen (e.g. fluorine, chlorine, bromine, iodine) and lower(C₁₋₄)alkoxy groups (e.g. methoxy, ethoxy, propoxyl, isopropoxy, butoxy, t-butoxy, etc.). Said "aryl group that may be substituted" for R₂ and R₃ include monocyclic and fused polycyclic aromatic hydrocarbon groups, which are exemplified by phenyl, naphthyl, anthryl, phenanthryl, acenaphthylenyl, etc. Preferably, said aryl group is phenyl group.

The substituent for said aryl group that may be substituted as designated by R₂ and R₃ includes but is not limited to halogens (e.g. fluorine, chlorine, bromine, iodine), lower alkyl groups that may be substituted, lower alkoxy groups that may be substituted, hydroxyl groups that may be substituted, nitro, and cyano, and said phenyl groups may be substituted by 1-3 (preferably 1-2) substituents which may be the same or different. The lower alkyl group mentioned above includes C₁₋₄ alkyl groups such as methyl, ethyl, n-propyl, isopropyl, n-butyl, isobutyl, sec-butyl, and tert-butyl and is preferably methyl or ethyl. The lower alkoxy group, also mentioned above, includes C₁₋₄ alkoxy groups such as methoxy, ethoxy, n-propoxy, isopropoxy, n-butoxy, isobutoxy, sec-butoxy, and tert-butoxy and is preferably methoxy or ethoxy. The substituent for said lower alkyl that may be substituted or for said lower alkoxy that may be substituted includes halogen (e.g. fluorine, chlorine, bromine, iodine), among others, and 1 to 5 substituents may be present in any possible positions. The substituent for said hydroxyl that may be substituted includes but is not limited to lower(C₁₋₄)alkyl (e.g. methyl, ethyl, propyl, isopropyl, butyl, t-butyl, etc.), C₃₋₆ cycloalkyl (e.g. cyclopropyl, cyclobutyl, cyclopentyl, cyclohexyl, etc.), C₆₋₁₀ aryl (e.g. phenyl, 1-naphthyl, 2-naphthyl, etc.), and C₇₋₁₂ aralkyl (e.g. benzyl, phenethyl, etc.). These substituents, located adjacent to each other, may form a ring system such as the following: 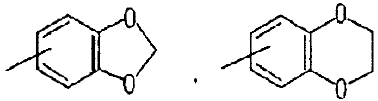 when the aryl group represented by R₂ or R₃ is a phenyl group.
The ring system thus formed may further have 1 to 4 substituents, exemplified by lower(C₁₋₃)alkyl (e.g. methyl, ethyl, propyl, isopropyl, etc.), among other groups.

The aromatic heterocyclic group of said heterocyclic group that may be substituted as designated by R₂ and R₃ includes the heterocyclic groups mentioned as the substituent of the hydrocarbon group for R₁ and is preferably a 5- or 6-membered aromatic monocyclic heterocyclic group such as furyl, thienyl, indolyl, isoindolyl, pyrazinyl, pyridyl, pyrimidyl, or imidazolyl. Said heterocyclic group may have 1 to 4 substituent(s) and the substituent for said heterocyclic group includes C₁₋₃ alkyl (e.g. methyl, ethyl, propyl, etc.), among other groups.

Among the groups mentioned above for R₂ and R₃, phenyl that may be substituted is preferred and phenyl substituted by halogen (e.g. chlorine, bromine), lower (C₁₋₃) alkoxy and/or the like is particularly preferred. Moreover, either R₂ or R₃ is preferably hydrogen.

Referring to formula (I), said substituent comprising carboxyl that may be esterified as designated by X' includes carboxyl that may be esterified and substituents having carboxyl that may be esterified. The carboxyl that may be esterified includes the groups mentioned hereinafter for the "carboxyl that may be esterified" as designated by Y

The substituent comprising carbamoyl that may be substituted as designated by X' includes carbamoyl that may be substituted and substituents having carbamoyl that may be substituted. The carbamoyl that may be substituted includes those groups mentioned hereinafter for the "carbamoyl that may be substituted" as designated by Y

The substituent comprising hydroxyl that may be substituted as designated by X' includes hydroxyl that may be substituted and substituents having hydroxyl that may be substituted. The hydroxyl that may be substituted includes the groups mentioned hereinafter for the "hydroxyl that may be substituted" as designated by Y

The substituent comprising amino that may be substituted as designated by X' includes amino that may be substituted and substituents having amino that may be substituted. The amino that may be substituted includes the groups mentioned hereinafter for the "amino that may be substituted" as designated by Y

The substituent comprising a substituted or unsubstituted heterocyclic group having a hydrogen atom that may be deprotonated namely having an active proton as designated by X' includes substituted or unsubstituted heterocyclic groups having a hydrogen atom that may be deprotonated and substituents having a substituted or unsubstituted heterocyclic group having a hydrogen atom that may be deprotonated. The substituted or unsubstituted heterocyclic group mentioned above includes the groups mentioned hereinafter for the "substituted or unsubstituted heterocyclic group having a hydrogen atom that may be deprotonated" as designated by Y

Thus, X' may, for example, be a group of the following formula (a).

.̅.̅.̅.̅.̅.̅.̅.̅ X-Y

wherein X represents a bond or a divalent or trivalent atomic chain; Y represents carboxyl that may be esterified, carbamoyl that may be substituted, hydroxyl that may be substituted, amino that may be substituted, or a substituted or unsubstituted heterocyclic group having a hydrogen atom that may be deprotonated; the broken line represents a single bond or a double bond.

The divalent atomic chain designated by X in formula (a) is preferably a divalent chain whose straight-chain moiety is composed of preferably 1-7 atoms and more preferably 1-4 atoms, and may have a side chain.

An example is 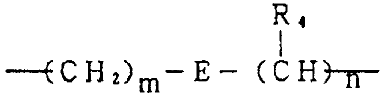 wherein m and n independently represent 0, 1, 2 or 3; E represents either a bond or an oxygen atom, a sulfur atom, sulfoxide, sulfone, -N(R₅)-, -NHCO-, -CON(R₆)- or -NHCONH-. Here, R₄ and R₆ each represents hydrogen, a lower alkyl group that may be substituted, an aralkyl group that may be substituted, or a phenyl group that may be substituted; R₅ represents hydrogen, a lower alkyl group, an aralkyl group or an acyl group.

The alkyl group of said lower alkyl group that may be substituted as designated by R₄ and R₆ includes but is not limited to straight-chain or branched lower(C₁₋₆)alkyl groups (e.g. methyl, ethyl, n-propyl, isopropyl, n-butyl, isobutyl, tert-butyl, n-pentyl, isopentyl, neopentyl, etc.). Said lower alkyl group may have 1 to 4 (preferably 1 to 2) substitutents. The substituent for said lower alkyl that may be substituted includes aromatic heterocyclic groups (e.g. 5 to 6 membered aromatic heterocyclic radical containing 1 to 4 hetero atoms selected from the group consisting of N, O and S such as furyl, thienyl, indolyl, isoindolyl, pyrazinyl, pyridyl, pyrimidyl, imidazolyl, etc.), amino that may be substituted (e.g. amino group or mono- or di substituted amino group etc.), hydroxyl that may be substituted, thiol that may be substituted, carboxyl that may be esterified, and halogen (e.g. fluorine, chlorine, bromine, iodine). The substituent for said amino that may be substituted, for said hydroxyl that may be substituted, or for said thiol that may be substituted includes lower(C₁₋₃)alkyl (e.g. methyl, ethyl, propyl, etc.), among other groups. The carboxyl that may be esterified includes C₂₋₅ alkoxy carbonyl and C₇₋₁₁ aryloxy carbonyl such as methoxycarbonyl, ethoxycarbonyl, propoxycarbonyl, phenoxycarbonyl, 1-naphthoxycarbonyl, etc. and is preferably methoxycarbonyl, ethoxycarbonyl or propoxycarbonyl.

The aralkyl group of said aralkyl that may be substituted as designated by R₄ and R₆ includes C₇-C₁₅ aralkyl groups such as benzyl, naphthylmethyl, phenylpropyl and phenylbutyl, among others. Said aralkyl group may have 1 to 4 (preferably 1 to 2) substituents. The substituent for said aralkyl group that may be substituted includes halogen (e.g. fluorine, chlorine, bromine, iodine), C₁₋₃ alkoxy (e.g. methoxy, ethoxy, propoxy, etc.), hydroxyl, amino, carboxyl and sulfhydryl, among others.

The substituent for said phenyl that may be substituted as designated by R₄ and R₆ includes halogen (e.g. fluorine, chlorine, bromine, iodine), C₁₋₃ alkoxy (e.g. methoxy, ethoxy, propoxy, etc.), and C₁₋₃ alkyl (e.g. methyl, ethyl, propyl, etc.), among others.

It should be understood that the substituent R₄ may vary with different methylene units.

The lower alkyl group and aralkyl group as designated by R₅ include lower(C₁₋₄)alkyl (e.g. methyl, ethyl, propyl, butyl, tert-butyl, etc.) and C₇₋₁₅ aralkyl (e.g. benzyl, phenethyl, phenylpropyl, phenylbutyl, naphthylmethyl, etc.) respectively.

The acyl group designated by R₅ includes lower (C₁₋₆) alkanoyl (e.g. formyl, acetyl, propionyl, butyryl, isobutyryl, valeryl, isovaleryl, pivaloyl, hexanoyl, etc.), lower (C₃₋₇) alkenoyl (e.g. acryloyl, methacryloyl, crotonoyl, isocrotonoyl, etc.), C₄₋₇ cycloalkanecarbonyl (cyclopropanecarbonyl, cyclobutanecarbonyl, cyclopentanecarbonyl, cyclohexanecarbonyl, etc.), lower (C₁₋₄) alkanesulfonyl (e.g. mesyl, ethanesulfonyl, propanesulfonyl, etc.), aroyl (C₂₋₁₄) (e.g. benzoyl, p-toluoyl, 1-naphthoyl, 2-naphthoyl, etc.), C₆₋₁₀ aryl(lower)(C₂₋₄) alkanoyl (e.g. phenylacetyl, phenylpropionyl, hydratropoyl, phenylbutyryl, etc.), C₆₋₁₀ aryl(lower)(C₃₋₅) alkenoyl (e.g. cinnamoyl, atropoyl, etc.), and C₆₋₁₀ arenesulfonyl (e.g. benzenesulfonyl, p-toluenesulfonyl, etc.).

X may further be a double bond-containing carbon chain or -L-CH(OH)- (L represents a bond or a straight-chain or branched alkylene chain). The double bond-containing carbon chain mentioned just above is preferably a carbon chain whose straight-chain moiety is composed of preferably 1 to 7 and more preferably 1 to 4 carbon atoms and may have a side chain. The double bond in said carbon chain may occur in either the straight-chain moiety or the branched chain or in both but is preferably present in the straight-chain moiety. There is no particular limitation on the number of double bonds present in the carbon chain but the presence of 1 to 2 double bonds is preferred.

The double bond-containing carbon chain includes methine, vinylene, propenylene, butenylene, butadienylene, methylpropenylene, ethylpropenylene, propylpropenylene, methylbutenylene, ethylbutenylene, propylbutenylene, methylbutadienylene, ethylbutadienylene, propylbutadienylene, pentenylene, hexenylene, heptenylene, pentadienylene, hexadienylene, heptadienylene, etc., with methine, vinylene, propenylene, butenylene and butadienylene being preferred. Where the carbon chain is trivalent, it is bound, in the manner of a double bond, to a substitutable ring carbon atom of ring J'.

The straight-chain or branched alkylene chain designated by L includes but is not limited to straight-chain or branched C₁₋₆ alkylene chains. Thus, for example, such divalent groups as methylene, ethylene, trimethylene, tetramethylene, pentamethylene, hexamethylene, heptamethylene, propylene, ethylmethylene, ethylethylene, propylethylene, butylethylene, methyltetramethylene, methyltrimethylene, etc. can be mentioned. Preferred are C₁₋₃ alkylene chains such as methylene, ethylene, trimethylene and propylene.

Among the various species mentioned for X, X' is preferably a group of formula (b).

-X₁-Y

wherein X₁ represents a bond or a divalent atomic chain; Y represents a carboxyl group that may be esterified, a carbamoyl group that may be substituted, a hydroxyl group that may be substituted, an amino group that may be substituted, or a substituted or unsubstituted heterocyclic group having a hydrogen atom that may be deprotonated.

The divalent atomic chain X₁ in formula (b) may be the same divalent atomic chain as mentioned hereinbefore for X.

Referring to formulas (a) and (b), the divalent atomic chain X or X₁ is preferably a straight-chain or branched alkylene chain whose straight-chain moiety is composed of 1 to 7 (more preferably 1 to 4) carbon atoms. The alkylene chain thus includes such divalent groups as methylene, ethylene, trimethylene, tetramethylene, pentamethylene, hexamethylene, heptamethylene, propylene, ethylmethylene, ethylethylene, propylethylene, butylethylene, methyltetramethylene, methyltrimethylene, etc. and is preferably a C₁₋₄ alkylene chain such as methylene, ethylene, trimethylene, and propylene, among others.

In formulas (a) and (b), said carboxyl group that may be esterified as designated by Y includes lower (C₂₋₇) alkoxycarbonyl (e.g. methoxycarbonyl, ethoxycarbonyl, propoxycarbonyl, isopropoxycarbonyl, butoxycarbonyl, tert-butoxycarbonyl, sec-butoxycarbonyl, pentyloxycarbonyl, iso-pentyloxycarbonyl, neopentyloxycarbonyl, etc.) and C₇₋₁₄ aryloxycarbonyl (e.g. phenoxycarbonyl, 1-naphthoxycarbonyl, C₈₋₁₂ aralkyl carbonyl (e.g. benzyloxycarbonyl, etc.). Particularly preferred are carboxyl, methoxycarbonyl and ethoxycarbonyl.

The substituent for said "carbamoyl group that may be substituted" as designated by Y includes lower(C₁₋₆)alkyl (e.g. methyl, ethyl, n-propyl, isopropyl, butyl, isobutyl, sec-butyl, tert-butyl, pentyl, isopentyl, neopentyl, hexyl, isohexyl, etc.) that may be substituted , C₃₋₆ cycloalkyl (e.g. cyclopropyl, cyclobutyl, cyclopentyl, cyclohexyl, etc.) that may be substituted, C₆₋₁₄ aryl (e.g. phenyl, 1-naphthyl, 2-naphthyl, etc.) that may be substituted, and C₇₋₁₁ aralkyl (e.g. benzyl, phenethyl, etc.) that may be substituted. These substituents for the carbamoyl group may have one or two substituent(s) which may be the same or different. The substituent for said lower(C₁₋₆)alkyl that may be substituted or for said C₃₋₆ cycloalkyl that may be substituted includes carboxyl that may be esterified by lower(C₁₋₅)alkyl (e.g. methyl, ethyl, propyl, isopropyl, butyl, t-butyl, pentyl, isopentyl, neopentyl, etc.), 5- or 6- membered aromatic heterocyclic groups containing 1 to 4 hetero atoms (e.g. furyl, thienyl, indolyl, isoindolyl, pyrazinyl, pyridyl, pyrimidyl, imidazolyl, etc.), amino, hydroxyl, and phenyl, among others, and 1 to 3 of such substituents, which may be the same or different, may be present. The substituent for said aryl that may be substituted and for said aralkyl that may be substituted include halogen (e.g. fluorine, chlorine, bromine, iodine) and carboxyl that may be esterified by lower(C₁₋₄)alkyl (e.g. methyl, ethyl, propyl, isopropyl, butyl, t-butyl, etc.), among others. The two substituents on the nitrogen atom in said carbamoyl group that may be substituted may, joined together with the N atom, form a cycloamino (cyclic amino) group and 1-azetidinyl, 1-pyrrolidinyl, piperidino, morpholino, thiomorpholino, and 1-piperazinyl may be mentioned as examples of said cycloamino.

The substituent for said "hydroxyl that may be substituted" as designated by Y includes but is not limited to lower(C₁₋₄)alkyl (e.g. methyl, ethyl, propyl, isopropyl, butyl, t-butyl, etc.), C₃₋₆ cycloalkyl (e.g. cyclopropyl, cyclobutyl, cyclopentyl, cyclohexyl, etc.), C₆₋₁₀ aryl (e.g. phenyl, 1-naphthyl, 2-naphthyl, etc.) that may be substituted, and C₇₋₁₁ aralkyl (e.g. benzyl, phenethyl, etc.) that may be substituted. The substituent for said aryl that may be substituted or for said aralkyl that may be substituted includes halogen (e.g. fluorine, chlorine, bromine, iodine) and carboxyl that may be esterified by lower(C₁₋₄)alkyl (e.g. methyl, ethyl, propyl, isopropyl, butyl, t-butyl, etc.), among others.

Said "amino that may be substituted" as designated by Y includes mono substituted and di substituted amino group. The substituent for said "amino that may be substituted" as designated by Y includes but is not lower(C₁₋₄)alkyl (e.g. methyl, ethyl, propyl, isopropyl, butyl, t-butyl, etc.), C₃₋₆ cycloalkyl (e.g. cyclopropyl, cyclobutyl, cyclopentyl, cyclohexyl, etc.), C₆₋₁₀ aryl (e.g. phenyl, 1-naphthyl, 2-naphthyl, etc.) that may be substituted, and C₇₋₁₁ aralkyl (e.g. benzyl, phenethyl, etc.) that may be substituted. The substituent for said aryl that may be substituted or for said aralkyl that may be substituted include 1 to 4 (preferably 1 to 2) substituents selected from the group consisting of halogen (e.g. fluorine, chlorine, bromine, iodine) and carboxyl that may be esterified by lower(C₁₋₄)alkyl (e.g. methyl, ethyl, propyl, isopropyl, butyl, t-butyl, etc.). The two substituents on the nitrogen atom in said amino group that may be substituted may, joined together with the N atom, form a cycloamino group and examples of such cycloamino group are 1-azetidinyl, 1-pyrrolidinyl, piperidino, morpholino and 1-piperazinyl, among others.

The heterocyclic group of said "substituted or unsubstituted heterocyclic group having a hydrogen atom that may be deprotonated" as designated by Y includes 5-7-membered (preferably 5-membered) monocyclic heterocyclic groups containing at least one member selected from among N, S and O (preferably nitrogen-containing heterocyclic groups) having a hydrogen atom that may be deprotonated, namely having an active proton. Thus, for example, tetrazol-5-yl and groups of the formula 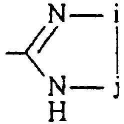 wherein i represents -O- or -S-; j represents >C=O, >C=S, or >S(O)₂ (inter alia, 2,5-dihydro-5-oxo-1,2,4-oxadiazol-3-yl, 2,5-dihydro-5-thioxo-1,2,4-oxadiazol-3-yl, and 2,5-dihydro-5-oxo-1,2,4-thiadiazol-3-yl are preferred).

The heterocyclic group mentioned above may be protected by a lower alkyl group (preferably C₁₋₄ alkyl) that may be substituted or an acyl group. The lower alkyl group that may be substituted includes C₁₋₄ alkyl group optionally substituted with phenyl which may be substituted by C₁₋₃ alkyl, nitro or C₁₋₃ alkoxy, or C₁₋₃ alkoxy such as ethyl, triphenylmethyl, methoxymethyl, ethoxymethyl, p-methoxybenzyl and p-nitrobenzyl, among others. The acyl group includes lower(C₂₋₅)alkanoyl, benzoyl and so on.

Among the various species of X', an alkyl group substituted by a carboxyl group that may be esterified, an alkyl group substituted by a substituted or unsubstituted heterocyclic group having a hydrogen atom that may be deprotonated or an alkyl group substituted by a carbamoyl group that may be substituted is preferred.

Referring to formula (I), the heterocyclic ring designated by ring A includes the "heterocyclic groups" as mentioned as the substituent of the hydrocarbon group for R₁ and is preferably a group of the following formula.

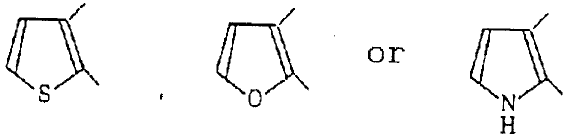

The substituent for said benzene ring that may be substituted or for said heterocyclic ring that may be substituted includes halogen (e.g. fluorine, chlorine, bromine, iodine), lower(C₁₋₄)alkyl (e.g. methyl, ethyl, propyl, butyl, tert-butyl, etc.) that may be substituted, lower(C₁₋₄)alkoxy (e.g. methoxy, ethoxy, propoxy, isopropoxy, butoxy, tert-butoxy, etc.) that may be substituted, hydroxyl, nitro, and cyano, among others. Ring A may have 1 to 3, preferably 1 to 2, of such substituents. These substituents, located adjacent to each other, may form a ring therebetween. The substituent for said lower alkyl that may be substituted or for said lower alkoxy that may be substituted includes halogen (e.g. fluorine, chlorine, bromine, iodine) and 1 to 3 of such substituents may be present in desired positions. The substituent on ring A is preferably methoxy or chlorine and more desirably chlorine.

Referring to formula (I'), the substituent for said "benzene ring that may be substituted" as designated by ring B includes halogen (fluorine, chlorine, bromine, iodine), lower(C₁₋₄)alkyl (e.g. methyl, ethyl, propyl, butyl, tert-butyl, etc.) that may be substituted, lower(C₁₋₄)alkoxy (e.g. methoxy, ethoxy, propoxy, isopropoxy, butoxy, tert-butoxy, etc.) that may be substituted, hydroxyl, nitro and cyano, among others. Ring B may have 1 to 3, preferably 1 to 2, of such substituents. Moreover, these substituents, located adjacent to each other, may form a ring therebetween. The substituent for said lower alkyl that may be substituted or for said lower alkoxy that may be substituted includes halogen (e.g. fluorine, chlorine, bromine, iodine) and may be present in 1 to 3 desired positions. The substituent on ring B is preferably methoxy or chlorine and more preferably chlorine.

Referring to formula (I), the heterocycle of said 7 or 8-membered heterocyclic ring containing at most 3 hetero-atoms as ring members as designated by ring J' includes 7 or 8-membered saturated or unsaturated heterocycles containing at least one member selected from among O, S(O)q (q is equal to 0, 1 or 2) and N. It should be understood, however, that the number of hetero-atoms constituting the ring (ring atoms) is not greater than 3.

In addition to the groups R₁, R₂, R₃ and X', ring J' may have 1 to 2 substituents in its substitutable positions. Among such substituents, the substituent attached to a ring nitrogen atom of ring J' includes alkyl (e.g. C₁₋₆ alkyl such as methyl, ethyl, n-propyl, isopropyl, n-butyl, isobutyl, tert-butyl, n-pentyl, isopentyl, neopentyl, etc.) and acyl (e.g. C₁₋₄ acyl such as formyl, acetyl, propionyl, butyroyl, etc.), among others. The alkyl or acyl mentioned just above may be substituted by halogen (e.g. fluorine, chlorine, bromine, iodine) in 1-5 positions. Where the substituent is attached to a ring carbon atom of ring J', it may for example be oxo, thioxo, hydroxyl that may be substituted, or amino that may be substituted. The hydroxyl that may be substituted and the amino that may be substituted, mentioned just above, may be the same as the hydroxyl that may be substituted and the amino that may be substituted, respectively, as defined hereinbefore for Y

In addition to the groups R₁, R₂, R₃ and X', ring J' is preferably substituted by oxo or thioxo in a substitutable position.

The fused ring composed of ring A and ring J' may for example be any of the following.

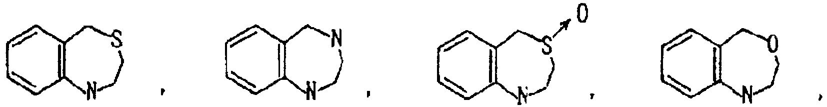

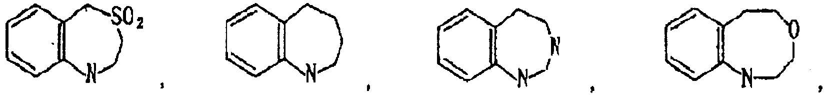

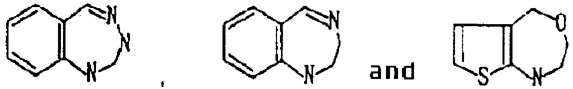

The preferred species of formula (I) is represented by the following formula (Ia): 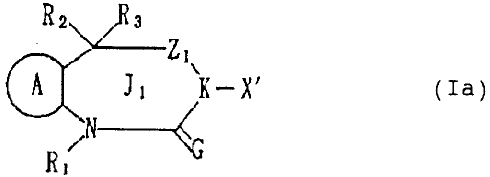 wherein R₁, R₂, R₃, X', and ring A are as defined hereinbefore; ring J' represents a 7-membered heterocycle; Z₁ represents -N(R₇)- (R₇ represents hydrogen, alkyl or acyl), -S(O)q- (q designates 0, 1 or 2), -CH₂- or -O-; K represents C or N; G represents O or S.

Referring to the above formula (Ia), the alkyl as represented by R₇ is a straight-chain or branched lower(C₁₋₆)alkyl group (e.g. methyl, ethyl, n-propyl, isopropyl, n-butyl, isobutyl, tert-butyl, n-pentyl, isopentyl, neopentyl, etc.), which may be substituted by 1 to 5 substituent(s) such as halogen (e.g. fluorine, chlorine, bromine, iodine).

The acyl R₇ may be a C₁₋₄ acyl group (e.g. formyl, acetyl, propionyl, butyryl, etc.) which may be substituted by 1 to 5 substituents(s) such as halogen (e.g. fluorine, chlorine, bromine, iodine).

In formula (Ia), Z₁ is preferably S(O)q (q = 0, 1 or 2) or O. Also preferred is C for K, and O for G.
Among compounds of formula (Ia), those of formula (Ib) are still more preferred. 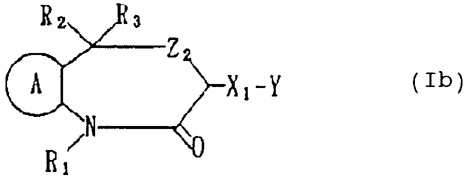 wherein R₁, R₂, R₃, X₁, Y, and ring A are as defined hereinbefore; Z₂ represents S(O)q (q = 0, 1 or 2) or O.

Referring to formula (I), the species of formula (I') are preferred.

Referring to formula (I'), the species of formula (I") are still more desirable. 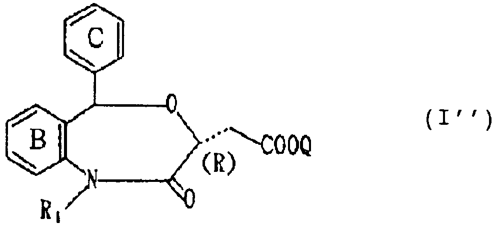 wherein R₁ and ring B are as defined hereinbefore; Q represents hydrogen or a metal ion; ring C represents phenyl that may be substituted. In the formula, the substituents in 3- and 5-positions are trans-oriented with respect to the plane of the 7-membered ring and (R) stands for R-configuration.

In the above formula (I"), the metal ion Q includes sodium ion, potassium ion, calcium ion, and aluminum ion, among others. Preferred are sodium ion and potassium ion.

The substituent of the phenyl that may be substituted as designated by ring C may be the same as the substituent for the aryl group that may be substituted as mentioned as an example of the hydrocarbon group that may be substituted defined for R₂ and R₃.

The salts of the compounds represented by the formulas (I), (Ia), (Ib), (I') and (I") include pharmacologically acceptable salts, for example salts with inorganic acids such as hydrochloric acid, hydrobromic acid, sulfuric acid, phosphoric acid, etc., salts with organic acids such as acetic acid, tartaric acid, citric acid, fumaric acid, maleic acid, toluenesulfonic acid, methanesulfonic acid, etc., salts with metals such as sodium, potassium, calcium, aluminum, etc., and salts with bases such as triethylamine, guanidine, ammonium, hydrazine, quinine and cinchonine, among others. Particularly, sodium salts are preferred.

The specific examples of the compounds of the present invention are as follows:
(3R,5S)-7-Cyano-5-(2,3-dimethoxyphenyl)-1-neopentyl-2-oxo-1,2,3,5-tetrahydro-4,1-benzoxazepine-3-acetic acid
(3R,5S)-7-Cyano-5-(2,4-dimethoxyphenyl)-1-neopentyl-2-oxo-1,2,3,5-tetrahydro-4,1-benzoxazepine-3-acetic acid
(3R,5S)-7-Cyano-5-(2,3-methylenedioxyphenyl)-1-neopentyl-2-oxo-1,2,3,5-tetrahydro-4,1-benzoxazepine-3-acetic acid
(3R,5S)-7-Cyano-5-(2,3-ethylenedioxyphenyl)-1-neopentyl-2-oxo-1,2,3,5-tetrahydro-4,1-benzoxazepine-3-acetic acid
(3R,5S)-7-Cyano-5-(2,3-dimethoxyphenyl)-1-isobutyl-2-oxo-1,2,3,5-tetrahydro-4,1-benzoxazepine-3-acetic acid
(3R,5S)-7-Cyano-5-(2,4-dimethoxyphenyl)-1-isobutyl-2-oxo-1,2,3,5-tetrahydro-4,1-benzoxazepine-3-acetic acid
(3R,5S)-7-Cyano-5-(2,3-methylenedioxyphenyl)-1-isobutyl-2-oxo-1,2,3,5-tetrahydro-4,1-benzoxazepine-3-acetic acid
(3R,5S)-7-Cyano-5-(2,3-ethylenedioxyphenyl)-1-isobutyl-2-oxo-1,2,3,5-tetrahydro-4,1-benzoxazepine-3-acetic acid
(3R,5S)-7-Chloro-5-(2,3-dimethoxyphenyl)-1-neopentyl-2-oxo-1,2,3,5-tetrahydro-4,1-benzoxazepine-3-acetic acid
(3R,5S)-7-Chloro-5-(2,4-dimethoxyphenyl)-1-neopentyl-2-oxo-1,2,3,5-tetrahydro-4,1-benzoxazepine-3-acetic acid
(3R,5S)-7-Chloro-5-(2,3-methylenedioxyphenyl)-1-neopentyl-2-oxo-1,2,3,5-tetrahydro-4,1-benzoxazepine-3-acetic acid
(3R,5S)-7-Chloro-5-(2,3-ethylenedioxyphenyl)-1-neopentyl-2-oxo-1,2,3,5-tetrahydro-4,1-benzoxazepine-3- acetic acid
(3R,5S)-7-Chloro-5-(2,3-dimethoxyphenyl)-1-isobutyl-2-oxo-1,2,3,5-tetrahydro-4,1-benzoxazepine-3-acetic acid
(3R,5S)-7-Chloro-5-(2,4-dimethoxyphenyl)-1-isobutyl-2-oxo-1,2,3,5-tetrahydro-4,1-benzoxazepine-3-acetic acid
(3R,5S)-7-Chloro-5-(2,3-methylenedioxyphenyl)-1-isobutyl-2-oxo-1,2,3,5-tetrahydro-4,1-benzoxazepine-3-acetic acid
(3R,5S)-7-Chloro-5-(2,3-ethylenedioxyphenyl)-1-isobutyl-2-oxo-1,2,3,5-tetrahydro-4,1-benzoxazepine-3-acetic acid
(3R,5S)-7-Cyano-5-(2,3-dimethoxyphenyl)-1-neopentyl-2-oxo-1,2,3,5-tetrahydro-4,1-benzothiazepine-3-acetic acid
(3R,5S)-7-Cyano-5-(2,4-dimethoxyphenyl)-1-neopentyl-2-oxo-1,2,3,5-tetrahydro-4,1-benzothiazepine-3-acetic acid
(3R,5S)-7-Cyano-5-(2,3-methylenedioxyphenyl)-1-neopentyl-2-oxo-1,2,3,5-tetrahydro-4,1-benzothiazepine-3-acetic acid
(3R,5S)-7-Cyano-5-(2,3-ethylenedioxyphenyl)-1-neopentyl-2-oxo-1,2,3,5-tetrahydro-4,1-benzothiazepine-3-acetic acid
(3R,5S)-7-Cyano-5-(2,3-dimethoxyphenyl)-1-isobutyl-2-oxo-1,2,3,5-tetrahydro-4,1-benzothiazepine-3-acetic acid
(3R,5S)-7-Cyano-5-(2,4-dimethoxyphenyl)-1-isobutyl-2-oxo-1,2,3,5-tetrahydro-4,1-benzothiazepine-3-acetic acid
(3R,5S)-7-Cyano-5-(2,3-methylenedioxyphenyl)-1-isobutyl-2-oxo-1,2,3, 5-tetrahydro-4,1-benzothiazepine-3-acetic acid
(3R,5S)-7-Cyano-5-(2,3-ethylenedioxyphenyl)-1-isobutyl-2-oxo-1,2,3,5-tetrahydro-4,1-benzothiazepine-3-acetic acid
(3R,5S)-7-Chloro-5-(2,3-dimethoxyphenyl)-1-neopentyl-2-oxo-1,2,3,5-tetrahydro-4,1-benzothiazepine-3-acetic acid
(3R,5S)-7-Chloro-5-(2,4-dimethoxyphenyl)-1-neopentyl-2-oxo-1,2,3,5-tetrahydro-4,1-benzothiazepine-3-acetic acid
(3R,5S)-7-Chloro-5-(2,3-methylenedioxyphenyl)-1-neopentyl-2-oxo-1,2,3,5-tetrahydro-4,1-benzothiazepine-3-acetic acid
(3R,5S)-7-Chloro-5-(2,3-ethylenedioxyphenyl)-1-neopentyl-2-oxo-1,2,3,5-tetrahydro-4,1-benzothiazepine-3-acetic acid
(3R,5S)-7-Chloro-5-(2,3-dimethoxyphenyl)-1-isobutyl-2-oxo-1,2,3,5-tetrahydro-4,1-benzothiazepine-3-acetic acid
(3R,5S)-7-Chloro-5-(2,4-dimethoxyphenyl)-1-isobutyl-2-oxo-1,2,3,5-tetrahydro-4,1-benzothiazepine-3-acetic acid
(3R,5S)-7-Chloro-5-(2,3-methylenedioxyphenyl)-1-isobutyl-2-oxo-1,2,3,5-tetrahydro-4,1-benzothiazepine-3-acetic acid
(3R,5S)-7-Chloro-5-(2,3-ethylenedioxyphenyl)-1-isobutyl-2-oxo-1,2,3,5-tetrahydro-4,1-benzothiazepine-3-acetic acid
(3R,5S)-7-Cyano-5-(2-chlorophenyl)-1-neopentyl-2-oxo-1,2,3,5-tetrahydro-4,1-benzoxazepine-3-acetic acid
(3R,5S)-7-Cyano-5-(2-chlorophenyl)-1-isobutyl-2-oxo-1,2,3,5-tetrahydro-4,1-benzoxazepine-3-acetic acid
(3R,5S)-7-Chloro-5-(2-chlorophenyl)-1-neopentyl-2-oxo-1,2,3,5-tetrahydro-4,1-benzoxazepine-3-acetic acid
(3R,5S)-7-Chloro-5-(2-chlorophenyl)-1-isobutyl-2-oxo-1,2,3,5-tetrahydro-4,1-benzoxazepine-3-acetic acid
(3R,5S)-7-Cyano-5-(2-chlorophenyl)-1-neopentyl-2-oxo-1,2,3,5-tetrahydro-4,1-benzothiazepine-3-acetic acid
(3R,5S)-7-Cyano-5-(2-chlorophenyl)-1-isobutyl-2-oxo-1,2,3,5-tetrahydro-4,1-benzothiazepine-3-acetic acid
(3R,5S)-7-Chloro-5-(2-chlorophenyl)-1-neopentyl-2-oxo-1,2,3,5-tetrahydro-4,1-benzothiazepine-3-acetic acid
(3R,5S)-7-Chloro-5-(2-chlorophenyl)-1-isobutyl-2-oxo-1,2,3,5-tetrahydro-4,1-benzothiazepine-3-acetic acid
(3R,5S)-7-Chloro-5-(4-ethoxy-2-methoxyphenyl)-1-neopentyl-2-oxo-1,2,3,5-tetrahydro-4,1-benzoxazepine-3-acetic acid
(3R)-7-Chloro-5-(2-chlorophenyl)-2,3-dihydro-1-isobutyl-2-oxo-1H-1,4-benzodiazepine-3-acetic acid
(3R,5S)-7-Chloro-5-(2-chlorophenyl)-2,3,4,5-tetrahydro-1-isobutyl-2-oxo-1H-1,4-benzodiazepine-3-acetic acid
N-[(3R,5S)-7-Chloro-5-(2-chlorophenyl)-1-neopentyl-2-oxo-1,2,3,5-tetrahydro-4,1-benzothiazepine-3-acetyl]glycine
(3R,5S)-7-Chloro-5-(2-chlorophenyl)-3-dimethylaminocarbonylmethyl-1-neopentyl-2-oxo-1,2,3,5-tetrahydro-4,1-benzothiazepine
7-Chloro-5-(2-chlorophenyl)-1-isobutyl-2-oxo-2,3,4,5-tetrahydro-1H-[1]-benzazepine-3-acetic acid
(3R,5S)-7-Chloro-5-(2-chlorophenyl)-1-neopentyl-1,2,3,5-tetrahydro-2-thioxo-4,1-benzoxazepine-3-acetic acid
(3R,5S)-7-Chloro-5-(2-chlorophenyl)-1-neopentyl-2-oxo-1,2,3,5-tetrahydro-4,1-thieno[2,3-e]oxazepine-3-acetic acid
(3R,5S)-7-Chloro-5-(2-methoxyphenyl)-1-neopentyl-2-oxo-1,2,3,5-tetrahydro-4,1-thieno[2,3-e]oxazepine-3-acetic acid
(3R,5S)-7-Chloro-1-isobutyl-5-(2-methoxyphenyl)-2-oxo-1,2,3,5-tetrahydro-4,1-thieno[2,3-e]oxazepine-3-acetic acid
(3R,5S)-7-Chloro-5-(3-hydroxy-2-methoxyphenyl)-1-neopentyl-2-oxo-1,2,3,5-tetrahydro-4,1-benzothiazepine-3-acetic acid
(3R,5S)-7-Chloro-5-(4-hydroxy-2-methoxyphenyl)-1-neopentyl-2-oxo-1,2,3,5-tetrahydro-4,1-benzothiazepine-3-acetic acid
(3R,5S)-1-Chloro-5-(3-hydroxy-2-methoxyphenyl)-1-isobutyl-2-oxo-1,2,3,5-tetrahydro-4,1-benzothiazepine-3-acetic acid
(3R,5S)-7-Chloro-5-(4-hydroxy-2-methoxyphenyl)-1-isobutyl-2-oxo-1,2,3,5-tetrahydro-4,1-benzothiazepine-3-acetic acid
(3R,5S)-7-Chloro-5-(3-ethoxy-2-methoxyphenyl)-1-neopentyl-2-oxo-1,2,3,5-tetrahydro-4,1-benzothiazepine-3-acetic acid
(3R,5S)-7-Chloro-5-(4-ethoxy-2-methoxyphenyl)-1-neopentyl-2-oxo-1,2,3,5-tetrahydro-4,1-benzothiazepine-3-acetic acid
(3R,5S)-7-Chloro-5-(3-ethoxy-2-methoxyphenyl)-1-isobutyl-2-oxo-1,2,3,5-tetrahydro-4,1-benzothiazepine-3-acetic acid
(3R,5S)-7-Chloro-5-(4-ethoxy-2-methoxyphenyl)-1-isobutyl-2-oxo-1,2,3,5-tetrahydro-4,1-benzothiazepine-3-acetic acid
(3R,5S)-7-Chloro-5-(2-chloro-3-methoxyphenyl)-1-neopentyl-2-oxo-1,2,3,5-tetrahydro-4,1-benzothiazepine-3-acetic acid
(3R,5S)-7-Chloro-5-(2-chloro-4-methoxyphenyl)-1-neopentyl-2-oxo-1,2,3,5-tetrahydro-4,1-benzothiazepine-3-acetic acid
(3R,5S)-7-Chloro-5-(2-chloro-3-methoxyphenyl)-1-isobutyl-2-oxo-1,2,3,5-tetrahydro-4,1-benzothiazepine-3-acetic acid
(3R,5S)-7-Chloro-5-(2-chloro-4-methoxyphenyl)-1-isobutyl-2-oxo-1,2,3,5-tetrahydro-4,1-benzothiazepine-3-acetic acid
(3R,5S)-7-Chloro-5-(2-chloro-3-hydroxyphenyl)-1-neopentyl-2-oxo-1,2,3,5-tetrahydro-4,1-benzothiazepine-3-acetic acid
(3R,5S)-7-Chloro-5-(2-chloro-4-hydroxyphenyl)-1-neopentyl-2-oxo-1,2,3,5-tetrahydro-4,1-benzothiazepine-3-acetic acid
(3R,5S)-7-Chloro-5-(2-chloro-3-hydroxyphenyl)-1-isobutyl-2-oxo-1,2,3,5-tetrahydro-4,1-benzothiazepine-3-acetic acid
(3R,5S)-7-Chloro-5-(2-chloro-4-hydroxyphenyl)-1-isobutyl-2-oxo-1,2,3,5-tetrahydro-4,1-benzothiazepine-3-acetic acid and their pharmacologically acceptable salts.

The compounds represented by the formulas (I), (Ia), (Ib), (I') and (I") and their salts [hereinafter referred to, including the salts, briefly as compounds (I), (Ia), (Ib), (I') and (I") respectively] are disclosed in EPA 567026, WO 95/21834 (JP Application 15531/1994), EPA 645377 (JP Application 229159/1994), and EAP 645378 (JP Application 229160/1994), among others, and can be produced in accordance with the processes described therein.

The compounds (I), (Ia), (Ib), (I') and (I") according to the present invention have plasma triglyceride lowering activity with a low toxicity or safely and are therefore of value for the prophylaxis and treatment of hyperlipemia such as hypertriglyceridemia in mammalian animals (e.g. the mouse, rat, rabbit, dog, cat, cattle, swine, man). Moreover, these compounds are useful for the prophylaxis or treatment of nephropathy such as nephritis and nephrosis, atherosclerosis, ischemic diseases, myocardial infarction, angina pectoris, aneurysm, cerebral arteriosclerosis, peripheral arteriosclerosis, thrombosis, diabetes (e.g. insulin-resistant), pancreatic disorder, and restenosis following percutaneous coronary arterioplasty (PTCA).

Since the compounds (I), (Ia), (Ib), (I') and (I") have plasma triglyceride lowering activity, they can be used with advantage in the treatment or prophylaxis of hperlipemia associated with elevation of triglyceride concentration, such as hypertriglyceridemia.

The compound of general formula (I) is a plasma tri-glyceride lowering agent. Its biological properties suggest that compound (I) is especially suitable for the therapy and prophylaxis of hyperlipemia, particularly hypertriglyceridemia, hyperlipoproteinemia and hypercholesterolemia, associated atherosclerotic vascular diseases and secondary diseases such as coronary heart diseases, cerebral ischemia, dysbasia intermittens, and gangrene, among other diseases.

In the treatment of these diseases, compound (I) may be used singly or in combination with other drug substances inclusive of plasma lipid or cholesterol lowering agents. In such uses, compound (I) is preferably administered in peroral dosage forms or, where necessary, rectally in suppository forms. The following are some typical drugs that may be used in said combination therapy:
- fibrates [e.g. clofibrate, bezafibrate, gemfibrozil, etc.], nicotinic acid and its derivative and analogs [e.g. acipimox, probucol, etc.]
- bile acid-binding resins [cholestyramine, colestipol, etc.]
- cholesterol absorption inhibitors [sitosterols, neomycin, etc.]
- cholesterol biosynthesis inhibitors [e.g. HMG-COA reductase inhibitors such as lovastatin, simvastatin, pravastatin, etc.]
- squalene epoxidase inhibitors [e.g. NB-598 and analogs, etc.]

Among other active drugs that can be used in said combination therapy are 2,3-epoxyxqualene lanosterolcyclase, e.g. decalin derivatives, azadecalin derivatives and indan derivatives.

In addition, compound (I) is suitable for the treatment of diseases associated with hyperchylomicronemia, such as acute pancreatitis. As the pathogenic process of pancreatitis, some are of the opinion that chylomicrons cause microemboli in the pancreatic capillary vessels, while others advance the view that in the presence of hyperchylomicronemia, the plasma triglycerides are decomposed by pancreatic lipase and the resulting elevation of free fatty acid concentration results in intense local irritation. Having plasma triglyceride lowering activity, compound (I) of this invention provides for the cure of pancreatitis and can be used singly or in combination with known therapeutic agents. For the treatment of the above-mentioned disease, compound (I) can be administered orally or topically either as it is alone or in combination with known active substances. Such multiple-agent anti-enzyme therapy may include any of the following enzyme inhibitors:
- aprotinin [e.g. Trasylol]
- gabexate meaylate [e.g. FOY]
- nafamostat mesilate [e.g. Futhan]
- citicoline [e.g. Nicholin], and
- urinastatin [e.g. Miraclid], among others.

For relief from pain, antioholinergic drugs, non-narcotic analgesics, and narcotics can also be employed.

A further interesting indication of compound (I) may be secondary hyperlipemia. This condition may develop from diabetes, hypothyroidism, nephrotic syndrome and chronic renal failure. These diseases are associated with hyperlipemia with high frequency, and it is said that hyperlipemia in turn exacerbates these diseases, thus setting a vicious cycle to going. In view of its plasma lipid lowering activity, compound (I) is further suitable for the treatment and prevention of progression of said diseases. For such purposes, compound (I) can be administered alone or in combination with the following drugs.
- Antidiabetic drugs epalrentat [e.g. Kinedak], semisynthetic human insulin [e.g. Penfill], recombinant human insulin [e.g. Humulin], glyburide [e.g. Euglucon], gliclazide [e.g. Glimicron], glibenclamide [e.g. Daonil], xylometazoline [e.g. Novorin]?, insulin zine suspension (e.g. Monotard], insulin analogs, [e.g. Basen (Takeda)] acarbose [e.g. Glucobay], acetohexamide [e.g. Dimelin], tolbutamide [e.g. Rastinon], Bumilcon; 1-butyl-3-metanilylurea, glyclopyramide [e.g.

Deamelin-S], insulin injection [e.g. lszilin], etc.
- Therapeutic drugs for hypothyroidism: dried thyroid [e.g. Thyreoid], levothyroxine sodium [e.g. Thyradin-S], Liothyronine sodium [e.g. Thyronine, Thyronamin];
- Therapeutic drugs for nephrotic syndrome: for steroid therapy that is usually indicated as the therapy of first choice, prednisolone [e.g. Prednine], Prednisolone sodium succinate [e.g. Prednine for injection], methylprednisolone sodium succinate [e.g. Solumedrol, betamethasone [e.g. Rinderon], etc.
- In addition, for anticoagulant therapy, dipyridamole [e.g. Persantin], dilazep dihydrochloride [e.g. Comelian], etc. can be employed.
- For the treatment of chronic renal failure, diuretics such as furosemide [e.g. Lasix], bumetanide [e.g. Lunetoron], azosemide [e.g. Diart], and antihypertensive drugs, e.g. angiotensin converting enzyme inhibitors such as delapril hydrochloride, enalapril maleate [e.g. Renivace], Ca-channel blockers such as manidipine, alpha-adreergic antagonists, etc.

A still further use for compound (I) of this invention is inhibition of thrombus formation. Since the plasma triglyceride concentration is positively correlated with factor VII involved in blood coagulation and the intake of w-3 fatty acids results in a decrease in TG concentration and suppression of coagulation, it is generally thought that hypertriglyceridemia stimulates thrombus formation. Moreover, VLDL from hyperlipemic patients more remarkably enhances secretion of plasminogen activator inhibitors from the vascular endothelial cells, it is also suggested that TG lowers fibrinolytic capacities. Therefore, in consideration of its TG-lowering activity, compound (I) is considered to be suitable for the prophylaxis and therapy of thrombosis. For this purpose, compound (I) can be administered alone or preferably in combination with the following known drugs.
- Prophylactic and therapeutic drugs for thrombosis:
   - anticoagulants such as heparin sodium, heparin calcium, warfarin potassium [e.g. Warfarin],
   - thrombolytics (e.g. urokinase), and
   - antiplatelet drugs such as aspirin, sulfinpyrazone [e.g. Anturan], dipyridamole [e.g. Persantin], ticlopidine hydrochloride [e.g. Panaldine], cilostazol [e.g. Pletaal].

The compounds (I), (Ia), (Ib), (I') and (I") including salts thereof can be used orally or non-orally, e.g. by injection, infusion, inhalation, rectal administration, or local administration. They can be used as they are or as pharmaceutical compositions or dosage forms (e.g. powders, granules, tablets, pills, capsules, injections, syrups, emulsions, elixers, suspensions, solutions, etc.), and such compositions and dosage forms can be manufactured by using at least one species of the compound of the invention with or without a pharmacologically acceptable carrier, adjuvant, excipient, binder and/or diluent.

Pharmaceutical compositions can be processed into dosage forms by the established or conventional pharmaceutical procedures. The preparations in dosage forms of these pharmaceutical compositions can be produced by mixing and/or kneading the active ingredients with additives such as excipients, diluents and carriers. As used in this specification, the term "non-oral" means any of subcutaneous, intravenous, intramuscular, intraperitoneal, infusion, drip and other routes of administration. Injectable dosage forms, such as sterile aqueous suspensions or oil suspensions for injection can be prepared by the procedures known to those skilled in the art with the aid of a suitable dispersant or a wetting agent and a suspending agent. Such sterile dosage forms for injection may also be sterile injectable solutions or suspensions in nontoxic parentally administrable diluents or solvents, for example aqueous solutions. The vehicle or solvent that can be used includes water, Ringer's solution, and isotonic saline, among others. Further, sterile nonvolatile oils can also be used as the solvent or the suspension vehicle. For such purposes, all kinds of nonvolatile oils and fatty acids may prove useful and natural, synthetic or semisynthetic fatty oils or fatty acids and natural, synthetic and semisynthetic mono-, di- or triglycerides can also be employed.

Suppositories for rectal administration can be manufactured by mixing the active substance with a suitable non-irritant excipient or a carrier substance that is solid at ordinary temperature but liquid at the temperature within the bowel or melts in the rectum to release the active substance, typically caccao butter, polyethylene glycol and so on.

The solid dosage form for oral administration includes powders, granules, tablets, pills, capsules, etc. as formulated in the described manner. Preparations or pharmaceutical compositions in such dosage forms can be produced by mixing or kneading the active compound with at least one additive such as excipient or a carrier for example, sucrose, lactose, cellulose sugar, mannitol (e.g. D-mannitol), maltitol, dextran, starch (e.g. corn starch) and its derivatives, microcrystalline cellulose agar, alginates, chitins, chitosans, pectins, gum tragacanth, gum arabic, gelatin and its derivatives, collagen and its derivatives, casein, albumin, synthetic or semisynthetic polymers, and glycerides. Preparations in such dosage forms may further contain other conventional additives, for example inert diluents, lubricants such as magnesium stearate, preservatives such as parabens, sorbic acid, etc., antioxidants such as ascorbic acid, α-tocopherol, cysteine, etc., disintegrators (e.g. croscarmellose sodium), binders (e.g. hydroxypropylcellulose), thickeners, buffers, sweetening agents, flavors, perfumes and so on. Tablets and pills may be enteric-coated. Peroral liquid dosage forms are medicinally acceptable emulsions, syrups, elixers, suspensions, solutions, etc. and these may contain inert diluents which are commonly employed, such as water, and, if desired, additives (e.g. glucose, taurrocholic acid, Tween 80)
These peroral liquid dosage forms can be produced by a conventional method, for example, mixing an active substance with a diluent and, if desired, additives.

Depending on the dosage forms, the preparations for oral administration contain an active substance generally in an amount of 0.01-99 weight %, preferably 0.1-90 weight %, usually 0.5-50 weight %.

The dosage for any given clinical case or patient is determined in consideration of age, body weight, general condition, gender, diet, dosing schedule, administration method, expected excretion rate, treatment regimen (combination with other drugs), and the current severity of disease, among other factors.

The antihypertriglyceridemic composition compresing compounds (I), (Ia), (Ib), (I') and (I") of the present invention are of low toxicity and can therefore be used safely. While the daily dose is dependent on the patient's condition and body weight, species of active compound, route of administration, etc., the recommended daily dose for an adult weighing about 60 kg as an active ingredientfor hyperlipemia is about 1-500 mg, preferably about 10-200 mg, for oral administration and about 0.1-100 mg, preferably about 0.5-50 mg more preferably about 1-20 mg, for non-oral administration. No toxicity is expected within the above dose range.

The following examples are intended to describe the present invention in further detail and should by no means be construed as defining the scope of the invention.

The compounds used in the following examples, (3R,5S)-7-chloro-5-(2,3-dimethoxyphenyl)-1-neopentyl-2-oxo-1,2,3,5-tetrahydro-4,1-benzoxazepine-3-acetic acid (hereinafter referred to also as compound A'), its sodium salt (hereinafter referred to also as compound A) (3R,5S)-7-chloro-5-(2,4-dimethoxyphenyl)-1-neopentyl-2-oxo-1,2,3,5-tetrahydro-4,1-benzoxazepine-3-acetate (compound B'), its sodium salt (compound B), (3S,5S)-7-chloro-5-(2-chlorophenyl)-1-neopentyl-2-oxo-1,2,3,5-tetrahydro-4,1-benzoxazepine 3-acetic acid (compound C'), its sodium salt (compound C), (3R,5S)-7-chloro-5-(4-ethoxy-2-methoxyphenyl)-1-neopentyl-2-oxo-1,2,3,5-tetrahydro-4,1-benzoxazepine-3-acetic acid (compound D') and its sodium salt (compound D), were prepared in accordance with the methods discribed in EPA567026 and WO 95/21834 as mentioned above. For example, compound A was prepared by obtaining the optically active isomer in accordance with Example 2 described in WO 95/21834 from the compound 12 which was obtained by hydrolysis of the compound 11 in Example 72 in EPA567026 in accordance with, Example 74, followed by convension into its sodium salt in accordance with Example 5 in WO 95/21834.

The other compounds A', B, B', C, C', D and D' were obtained in the same manner.

### Examples

### Formulation Examples

A variety of dosage forms for the treatment of hyperlipemia can be manufactured using the compound (I), (Ia), (Ib), (I') or (I"), in accordance with the present invention by the following exemplary procedures.

### 1. Capsules

| | |
|---|---|
| (1) Sodium (3R,5S)-7-chloro-5-(2,3-dimethoxyphenyl)-1-neopentyl-2-oxo-1,2,3,5-tetrahydro-4,1-benzoxazepine-3-acetate (compound A) | 10 mg |
| (2) Lactose | 90 mg |
| (3) Microcrystalline cellulose | 70 mg |
| (4) Magnesium stearate | 10 mg |
| | 180 mg per capsule |

| | |
|---|---|
| (1), (2), (3) and 1/2 of (4) are blended and granulated. To the granulation is added the balance of (4), and the whole mixture is filled in a gelatin capsule shell. | |

### 2. Tablets

| | |
|---|---|
| (1) Sodium (3R,5S)-7-chloro-5-(2,3-dimethoxyphenyl)-1-neopentyl-2-oxo-1,2,3,5-tetrahydro-4,1-benzoxazepine-3-acetate (compound A) | 10 mg |
| (2) Lactose | 35 mg |
| (3) Corn starch | 150 mg |
| (4) Microcrystalline cellulose | 30 mg |
| (5) Magnesium stearate | 5 mg |
| | 230 mg per tablet |

| | |
|---|---|
| (1), (2), (3), 2/3 of (4), and 1/2 of (5) are blended and granulated. To the granulation is added the balance of (4) and (5), and the whole mixture is compressed into a tablet. | |

### 3. Injection

| | |
|---|---|
| (1) Sodium (3R,5S)-7-chloro-5-(2,3-dimethoxyphenyl)-1-neopentyl-2oxo-1,2,3,5-tetrahydro-4,1-benzoxazepine-3-acetate (compound A) | 10 mg |
| (2) Inositol | 100 mg |
| (3) Benzyl alcohol | 20 mg |
| | 130 mg per ampule |

| | |
|---|---|
| (1), (2) and (3) are dissolved in distilled water for injection to make 2 ml and the solution is filled in an ampule. The whole procedure is followed aseptically. | |

### 4. Capsules

| | |
|---|---|
| (1) Sodium (3R,5S)-7-chloro-5-(2,4-dimethoxyphenyl)-1-neopentyl-2-oxo-1,2,3,5-tetrahydro-4,1-benzoxazepine-3-acetate (compound B) | 10 mg |
| (2) Lactose | 90 mg |
| (3) Microcrystalline cellulose | 70 mg |
| (4) Magnesium stearate | 10 mg |
| | 180 mg per capsule |

| | |
|---|---|
| (1), (2), (3) and 1/2 of (4) are blended and granulated. To the granulation is added the balance of (4) and the whole mixture is filled in a gelatin capsule shell. | |

### 5. Tablets

| | |
|---|---|
| (1) Sodium (3R,5S)-7-chloro-5-(2,4-dimethoxyphenyl)-1-neopentyl-2-oxo-1,2,3,5-tetrahydro-4,1-benzoxazepine-3-acetate (compound B) | 10 mg |
| (2) Lactose | 35 mg |
| (3) Corn starch | 150 mg |
| (4) Microcrystalline cellulose | 30 mg |
| (5) Magnesium stearate | 5 mg |
| | 230 mg per tablet |

| | |
|---|---|
| (1), (2), (3), 2/3 of (4) and 1/2 of (5) are blended and granulated. To the granulation is added the balance of (4) and (5) and the whole mixture is compressed into a tablet. | |

### 6. Injection

| | |
|---|---|
| (1) Sodium (3R,5S)-7-chloro-5-(2,4-dimethoxyphenyl)-1-neopentyl-2-oxo-1,2,3,5-tetrahydro-4,1-benzoxazepine-3-acetate (compound B) | 10 mg |
| (2) Inositol | 100 mg |
| (3) Benzyl alcohol | 20 mg |
| | 130 mg per ampule |

| | |
|---|---|
| (1), (2), and (3) are dissolved in distilled water for injection to make 2 ml and the solution is filled in an ampule. The whole procedure is followed aseptically. | |

### 7. Capsules

| | |
|---|---|
| (1) Sodium (3R,5S)-7-chloro-5-(4-ethoxy-2-methoxy-phenyl)-1-neopentyl-2-oxo-1,2,3,5-tetrahydro-4,1-benzoxazepine-3-acetate (compound D) | 10 mg |
| (2) Lactose | 90 mg |
| (3) Microcrystalline cellulose | 70 mg |
| (4) Magnesium stearate | 10 mg |
| | 180 mg per capsule |

| | |
|---|---|
| (1), (2), (3) and /12 of (4) are blended and granulated. To the granulation is added the balance of (4) and the whole mixture is filled in a gelatin capsule shell. | |

### 8. Tablets

| | |
|---|---|
| (1) Sodium (3R,5S)-7-chloro-5-(4-ethoxy-2-methoxy-phenyl)-1-neopentyl-2-oxo-1,2,3,5-tetrahydro-4,1-benzoxazepine-3-acetate (compound D) | 10 mg |
| (2) Lactose | 35 mg |
| (3) Corn starch | 150 mg |
| (4) Microcrystalline cellulose | 30 mg |
| (5) Magnesium stearate | 5 mg |
| | 230 mg per tablet |

| | |
|---|---|
| (1), (2), (3), 2/3 of (4), and 1/2 of (5) are blended and granulated. To the granulation is added the balance of (4) and (5) and the whole mxiture is compressed into a tablet. | |

### 9. Injection

| | |
|---|---|
| (1) Sodium (3R,5S)-7-chloro-5-(4-ethoxy-2-methoxyphenyl)-1-neopentyl-2-oxo-1,2,3,5-tetrahydro-4,1-benzoxazepine-3-acetate (compound D) | 10 mg |
| (2) Inositol | 100 mg |
| (3) Benzyl alcohol | 20 mg |
| | 130 mg per ampule |

| | |
|---|---|
| (1), (2), and (3) are dissolved in distileld water for injection to make 2 ml and the solution is filled in an ampule. The whole procedure is followed aseptically. | |

### 10. Capsules

| | |
|---|---|
| (1) (3R,5S)-7-Chloro-5-(2,3-dimethoxyphenyl)-l-neopentyl-2-oxo-1,2,3,5-tetrahydro-4,1-benzoxazepine-3-acetic acid (compound A') | 10 mg |
| (2) Lactose | 90 mg |
| (3) Microcrystalline cellulose | 70 mg |
| (4) Magnesium stearate | 10 mg |
| | 180 mg per capsule |

| | |
|---|---|
| (1), (2), (3), and 1/2 of (4) are blended and granulated. To the granulation is added the balance of (4) and the whole mixture is filled in a gelatin capsule shell. | |

### 11. Tablets

| | |
|---|---|
| (1) (3R,5S)-7-Chloro-5-(2,3-dimethoxyphenyl)-1-neopentyl-2-oxo-1,2,3,5-tetrahydro-4,1-benzoxazepine-3-acetic acid (compound A') | 10 mg |
| (2) Lactose | 35 mg |
| (3) Corn starch | 150 mg |
| (4) Microcrystalline cellulose | 30 mg |
| (5) Magnesium stearate | 5 mg |
| | 230 mg per tablet |

| | |
|---|---|
| (1), (2), (3), 2/3 of (4), and 1/2 of (5) are blended and granulated. To the granulation is added the balance of (4) and (5) and the whole mixture is compressed into a tablet. | |

### 12. Capsules

| | |
|---|---|
| (1) 3R,5S)-7-Chloro-5-(2,4-dimethoxyphenyl)-1-neopentyl-2-oxo-1,2,3,5-tetrahydro-4,1-benzoxazepine-3-acetic acid (compound B') | 10 mg |
| (2) Lactose | 90 mg |
| (3) Microcrystalline cellulose | 70 mg |
| (4) Magnesium stearate | 10 mg |
| | 180 mg per capsule |

| | |
|---|---|
| (1), (2), (3), and 1/2 of (4) are blended and granulated. To the granulation is added the balance of (4) and the whole mixture is filled in a gelatin capsule shell. | |

### 13. Tablets

| | |
|---|---|
| (1) (3R,5S)-7-Chloro-5-(2,4-dimethoxyphenyl)-1-neopentyl-2-oxo-1,2,3,5-tetrahydro-4,1-benzoxazepine-3-acetic acid (compound B') | 10 mg |
| (2) Lactose | 35 mg |
| (3) Corn starch | 150 mg |
| (4) Microcrystalline cellulose | 30 mg |
| (5) Magnesium stearate | 5 mg |
| | 230 mg per tablet |

| | |
|---|---|
| (1), (2), (3), 2/3 of (4), and 1/2 of (5) are blended and granulated. To the granulation is added the balance of (4) and (5) and the whole mixture is compressed into a tablet. | |

### 14. Capsules

| | |
|---|---|
| (1) (3R,5S)-7-Chloro-5-(4-ethoxy-2-methoxyphenyl)-1-neopentyl-2-oxo-1,2,3,5-tetrahydro-4,1-benzoxazepine-3-acetic acid (compound D') | 10 mg |
| (2) Lactose | 90 mg |
| (3) Microcrystalline cellulose | 70 mg |
| (4) Magnesium stearate | 10 mg |
| | 180 mg per capsule |

| | |
|---|---|
| (1), (2), (3) and 1/2 of (4) are blended and granulated. To the granulation is added the balance of (4) and the whole mixture is filled in a gelatin capsule shell. | |

### 15. Tablets

| | |
|---|---|
| (1) (3R,5S)-7-Chloro-5-(4-ethoxy-2-methoxyphenyl)-1-neopentyl-2-oxo-1,2,3,5-tetrahydro-4,1-benzoxazepine-3-acetic acid (compound D) | 10 mg |
| (2) Lactose | 35 mg |
| (3) Corn starch | 150 mg |
| (4) Microcrystalline cellulose | 30 mg |
| (5) Magnesium stearate | 5 mg |
| | 230 mg per tablet |

| | |
|---|---|
| (1), (2), (3), 2/3 of (4), and 1/2 of (5) are blended and granulated. To the granulation is added the balance of (4) adn (5) and the whoel mixture is compressed into a tablet. | |

### 16. Tablets

According to the formulation shown below, a mixture of 175 g of compound A, 175 g of D-mannitol, 118.65 g of corn starch, and 105 g of croscarmellose sodium was thoroughly blended with a vertical granulator (Model PM-VG-10, Powrex) and kneaded with an aqueous solution of 19.25 g of hydroxypropylcellulose (kneading conditions: 400 rpm, 10 min.). The white kneaded composition was dried in a fluidized-bed drier (Model FD-3S, Powrex) at a feed air temperature of 60°C for 30 minutes and, then, sieved through a 1.5 mm (dia.) punched screen using a power mill (Moidel P-3, Showa Chemical Machinery Works) to provide a granulation. To 525.14 g of the granulation were added 31 g of croscarmellose sodium and 1.86 g of magnesium stearate and the whole composition was blended using a mixer (Model TM-15, Showa Chemical Machinery Works) for 5 minutes to provide compression molding granules. The granules, 180 mg, were compressed with a tablet machine (Correct 19K, Kikusui Seisakusho) using a 8.0 mm (dia.) flat-corner punch at a pressure of 0.7 ton/cm² to provide 2,350 tablets.

| | |
|---|---|
| Compound A | 50 mg |
| D-Mannitol | 50 mg |
| Corn starch | 33.9 mg |
| Croscarmellose sodium | 40 mg |
| Hydroxypropylcellulose | 5.5 mg |
| Magnesium stearate | 0.6 mg |
| Total | 180.0 mg per tablet |

### Test Examples

The biological activities of the compounds (I), (Ia), (Ib), (I') and (I") according to the present invention are now described by way of test example.

### Test Example 1 (Plasma lipid concentration-lowering activity in marmosets)

[Method] By a stomach tube, an aqueous solution of sodium (3R,5S)-7-chloro-5-(2,3-dimethoxyphenyl)-1-neopentyl-2-oxo-1,2,3,5-tetrahydro-4,1-benzoxazepine-3-acetate (compound A), sodium (3R,5S)-7-chloro-5-(2,4-dimethoxyphenyl)-1-neopentyl-2-oxo-1,2,3,5-tetrahydro-4,1-benzoxazepine-3-acetate (compound B), or sodium (3R,5S)-7-chloro-5-(2-chlorophenyl)-1-neopentyl-2-oxo-1,2,3,5-tetrahydro-4,1-benzoxazepine-3-acetate (compound C) was administered to male Common marmosets (19-68 months old, purchased from Japan EDM) for 4 days. Under non-fasted condition, blood was taken from the femoral vein and the plasma total cholesterol, HDL (High-density lipoprotein)-cholesterol, and triglyceride were measured enzymatically using an assay kit (Wako Pure Chemical Industries). The non-HDL-cholesterol was calculated by subtracting HDL-cholesterol from total cholesterol.
[Results] The results are shown in Table 1.

**[Table 1]**

| Plasma lipid lowering activity in marmosets | | | | |
|---|---|---|---|---|
| Test compound | Compound A | | Compound B | Compound C |
| Dosage (mg/kg/day) | 10 | 50 | 50 | 50 |
| number of animals | 6 | 5 | 3 | 3 |
| Total cholesterol | 18±9* | 21± 16* | 21±5* | 3±3 |
| Non-HDL-cholesterol | 34±8* | 53±9* | 25±11* | 27±11* |
| Triglyceride | 25±13* | 59±16* | 64±10* | 44±17* |

The pretreatment baselines: total cholesterol = 169±29 mg/dI, non-HDL-cholesterol = 98±19 mg/dI, triglyceride = 253±177 mg/dI. The values shown represent percent decreases from the pretreatment baselines and are expressed in mean ± SD. The asterisk denotes a significant change (p<0.05) compared with the baseline by Student's paired t-test.

The plasma total cholesterol, non-HDL-cholesterol and triglyceride concentrations were significantly decreased by the test compounds in a dose-dependen manner.

### Test Example 2 (Plasma lipid lowering activity in Wistar fatty rats)

[Method] Using a stomach tube, a solution of sodium (3R,5S)-7-chloro-5-(2,3-dimethoxyphenyl)-1-neopentyl-2-oxo-1,2,3,5-tetrahydro-4,1-benzoxazepine-3-acetate (hereinafter referred to as the test compound) in 0.5% methylcellulose was administered to male Wistar fatty rats (30 weeks old; Ikeda, H., Shino, A., Matsuo, T, Iwatsuka, H., and Suzuoki, Z.: Diabetes, 30, 1045 (1981)) for 2 weeks. Under non-fasted condition, blood was drawn from the orbital venus plexus and the plasma total cholesterol and HDL-cholesterol were determined enzymatically using an assay kit (Wako Pure Chemical Industries). The non-HDL-cholesterol was calculated by subtracting HDL-cholesterol from total cholesterol. The triglyceride was determined enzymatically using an assay kit (latron Labratories Inc.).
[Results] The results are shown in Table 2.

**[Table 2]**

| Plasma lipid lowering activity in Wistar fatty rats | | | |
|---|---|---|---|
| Dosage (mg/kg/day) | Control 0 | Test compound | |
| | | 10 | 30 |
| number of animals | 6 | 6 | 6 |
| Total cholesterol (mg/dl) | 186±15^{a} | 173±17^{ab} | 160±25^{b} |
| Non-HDL-cholesterol (mg/dl) | 77.8±6.3^{a} | 70.8±11.6^{a} | 52.6±9.2^{b} |
| Triglycerides (mg/dl) | 353±68^{a} | 295±49^{a} | 230±30^{b} |

Each value is mean ±SD.
Different superscripts letters in the same row of the table indicate a significant difference (p<0.05) between the respective values by Duncan's multiple-range test.

The total cholesterol, non-HDL-cholesterol and triglyceride in plasma were significantly lowered by the test compound in a dose-dependent manner.

The compounds of general formulas (I), (Ia), (Ib), (I') and (I") according to the present invention have plasma triglyceride lowering activity and are, therefore, of value for the prophylaxis or treatment of hyperlipemia such as hypertriglyceridemia in mammalian animals (e.g. monkey mouse, rat, rabbit, dog, cat, cattle, swine, man).

## Claims

1. An antihypertriglyceridemic composition comprising a compound of the formula (I) 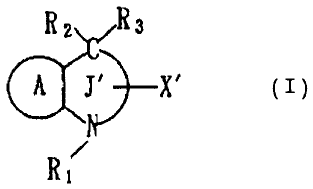 wherein R₁ represents a hydrogen atom or a hydrocarbon group that may be substituted; R₂ and R₃ may be the same or different and each represents a hydrogen atom, a hydrocarbon group that may be substituted, or a heterocyclic group that may be substituted; X' represents a substituent comprising a carboxyl group that may be esterified, a carbamoyl group that may be substituted, a hydroxyl group that may be substituted, an amino group that may be substituted, or an optionally substituted heterocyclic group having a hydrogen atom that may be deprotonated; ring A represents a benzene ring that may be substituted or a heterocyclic ring that may be substituted; ring J' represents a 7- or 8-membered heterocyclic ring having at most 3 hetero-atoms as ring constituent members; and ring J' may have a further substituent in addition to R₁, R₂, R₃ and X', or a pharmacologically acceptable salt thereof.

2. An antihypertriglyceridemic composition of Claim 1 wherein R₁ represents an aliphatic acyclic hydrocarbon group.

3. An antihypertriglyceridemic composition of Claim 2 wherein said aliphatic acyclic hydrocarbon group is a branched alkyl group.

4. An antihypertriglyceridemic composition of Claim 1 wherein R₂ or R₃ is a phenyl group that may be substituted.

5. An antihypertriglyceridemic composition of Claim 1 wherein X' represents an alkyl group substituted by a carboxyl group that may be esterified.

6. An antihypertriglyceridemic composition of Claim 1 wherein X' represents an alkyl group substituted by an optionally substituted heterocyclic group having a hydrogen atom that may be deprotonated.

7. An antihypertriglyceridemic composition of Claim 6 wherein said heterocyclic group is

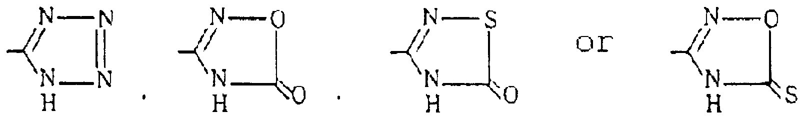

8. An antihypertriglyceridemic composition of claim 1 wherein X' represents an alkyl group substituted by a carbamoyl group that may be substituted.

9. An antihypertriglyceridemic composition of claim 8 wherein said carbamoyl group is a cyclic amino carbonyl group.

10. An antihypertriglyceridemic composition of Claim 5, 6 or 8 wherein said alkyl group is a straight-chain C₁₋₄ alkyl group.

11. An antihypertriglyceridemic composition of Claim 1 wherein said heterocyclic ring for ring A is

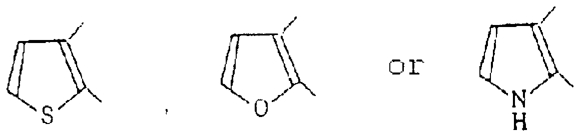

12. An antihypertriglyceridemic composition of Claim 1 wherein said further substituent on ring J' is oxo or thioxo.

13. An antihypertriglyceridemic composition of Claim 1 wherein the fused ring system consisting of ring A and ring J' is

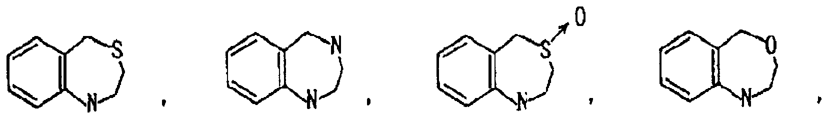

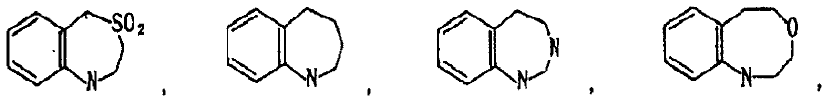

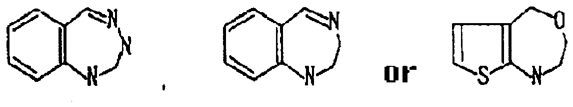

14. An antihypertriglyceridemic composition of Claim 1 wherein R₂ and R₃ are independently a hydrogen atom, an alkyl group that may be substituted, a phenyl group that may be substituted, or an aromatic heterocyclic group that may be substituted.

15. An antihypertriglyceridemic composition comprising a compound of the formula (I') 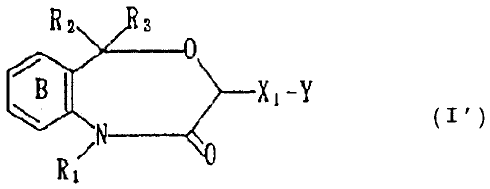 wherein R₁ represents a hydrogen atom or a hydrocarbon group that may be substituted; R₂ and R₃ may be the same or different and each represents a hydrogen atom, a hydrocarbon group that may be substituted, or a heterocyclic group that may be substituted; X₁ represents a bond or a divalent atomic chain; Y represents a carboxyl group that may be esterified, a carbamoyl group that may be substituted, a hydroxyl group that may be substituted, an amino group that may be substituted, or an optionally substituted heterocyclic group having a hydrogen atom that may be deprotonated and ring B represents a benzene ring that may be substituted, or a pharmacologically acceptable salt thereof.

16. An antihypertriglyceridemic composition of Claim 15 wherein R₁ represents an aliphatic acyclic hydrocarbon group.

17. An antihypertriglyceridemic composition of Claim 16 wherein said aliphatic acyclic hydrocarbon group is a branched alkyl group.

18. An antihypertriglyceridemic composition of Claim 15 wherein R₂ or R₃ is a phenyl group that may be substituted.

19. An antihypertriglyceridemic composition of Claim 15 wherein X₁ represents a straight-chain or branched alkylene chain.

20. An antihypertriglyceridemic composition of Claim 19 wherein said alkylene chain is a straight-chain C₁₋₄ alkylene chain.

21. An antihypertriglyceridemic composition of Claim 15 wherein Y is a carboxyl group that may be esterified.

22. An antihypertriglyceridemic composition of Claim 15 wherein Y is an optionally substituted heterocyclic group having a hydrogen atom that may be deprotonated.

23. An antihypertriglyceridemic composition of Claim 22 wherein said heterocyclic group is

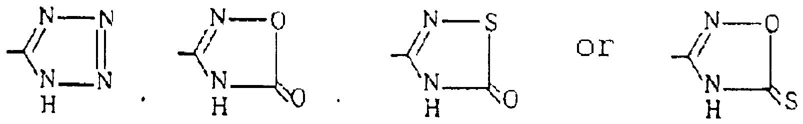

24. An antihypertriglyceridemic composition of Claim 1 comprising (3R,5S)-7-chloro-5-(2,3-dimethoxyphenyl)-1-neopentyl-2-oxo-1,2,3,5-tetrahydro-4,1-benzoxazepine-3-acetic acid or a pharmacologically acceptable salt thereof.

25. An antihypertriglyceridemic composition of Claim 1 comprising (3R,5S)-7-chloro-5-(2,4-dimethoxyphenyl)-1-neopentyl-2-oxo-1,2,3,5-tetrahydro-4,1-benzoxazepine-3-acetic acid or a pharmacologically acceptable salt thereof.

26. An antihypertriglyceridemic composition of Claim 1 comprising (3R,5S)-7-chloro-5-(4-ethoxy-2-methoxyphenyl)-1-neopentyl-2-oxo-1,2,3,5-tetrahydro-4,1-benzoxazepine-3-acetic acid or a pharmacologically acceptable salt thereof.

27. An antihypertriglyceridemic composition of Claim 24, 25 or 26 wherein said pharmacologically acceptable salt is a sodium salt.

28. An antihypertriglyceridemic composition of Claim 1 for the prophylaxis or treatment of hyperlipemia.

29. An antihypertriglyceridemic composition of Claim 1 for the prophylaxis or treatment of hypertriglyceridemia.

30. Use of a compound of the formula (I) 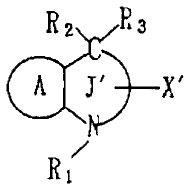 wherein R₁ represents a hydrogen atom or a hydrocarbon group that may be substituted; R₂ and R₃ may be the same or different and each represents a hydrogen atom, a hydrocarbon group that may be substituted, or a heterocyclic group that may be substituted; X' represents a substituent comprising a carboxyl group that may be esterified, a carbamoyl group that may be substituted, a hydroxyl group that may be substituted, an amino group that may be substituted, or an optionally substituted heterocyclic group having a hydrogen atom that may be deprotonated; ring A represents a benzene ring that may be substituted or a heterocyclic ring that may be substituted; ring J' represents a 7- or 8-membered heterocyclic having at most 3 hetero-atoms as ring constituent members; and ring J' may have a further substituent in addition to R₁, R₂, R₃ and X', or a pharmacologically acceptable salt thereof for the preparation of an antihypertriglyceridemic medicine for preventing or treating hypertriglyceridemia.

31. A method for prophylaxis or treatment of hypertriglyceridemia in a mammal which comprises administering to a subject in need an effective amount of a compound of the formula (I) 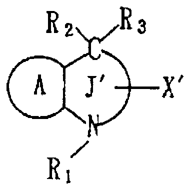 wherein R₁ represents a hydrogen atom or a hydrocarbon group that may be substituted; R₂ and R₃ may be the same or different and each represents a hydrogen atom, a hydrocarbon group that may be substituted, or a heterocyclic group that may be substituted; X' represents a substituent comprising a carboxyl group that may be esterified, a carbamoyl group that may be substituted, a hydroxyl group that may be substituted, an amino group that may be substituted, or an optionally substituted heterocyclic group having a hydrogen atom that may be deprotonated; ring A represents a benzene ring that may be substituted or a heterocyclic ring that may be substituted; ring J' represents a 7- or 8-membered heterocyclic having at most 3 hetero-atoms as ring constituent members; and ring J' may have a further substituent in addition to R₁, R₂, R₃ and X', or a pharmacologically acceptable salt thereof.

32. A method for producing an antihypertriglyceridemic composition comprising a compound of the formula (I) 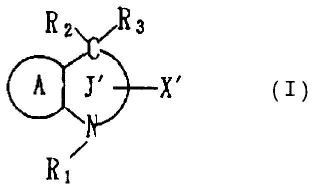 wherein R₁ represents a hydrogen atom or a hydrocarbon group that may be substituted; R₂ and R₃ may be the same or different and each represents a hydrogen atom, a hydrocarbon group that may be substituted, or a heterocyclic group that may be substituted; X' represents a substituent comprising a carboxyl group that may be esterified, a carbamoyl group that may be substituted, a hydroxyl group that may be substituted, an amino group that may be substituted, or an optionally substituted heterocyclic group having a hydrogen atom that may be deprotonated; ring A represents a benzene ring that may be substituted or a heterocyclic ring that may be substituted; ring J' represents a 7- or 8-membered heterocyclic having at most 3 hetero-atoms as ring constituent members; and ring J' may have a further substituent in addition to R₁, R₂, R₃ and X', or a pharmacologically acceptable salt thereof, which comprises mixing and/or kneading an effective amount of a compound of the formula (I) or a pharmacologically acceptable salt thereof with a pharmacologically acceptable diluent, excipient or carrier.
